# EUROPEAN PATENT APPLICATION

(11) **EP 2 209 075 A1**
(43) Date of publication of application: **21.07.2010**
(21) Application number: 08844737.0
(22) Date of filing: 31.10.2008
(51) Int. Cl.: G06F 19/00, C12M 1/00, C12Q 1/68, G06F 17/30, C12N 15/09

(54) **BASE SEQUENCE DETERMINATION PROGRAM, BASE SEQUENCE DETERMINATION SYSTEM AND BASE SEQUENCE DETERMINATION METHOD**

(30) Priority: 31.10.2007 JP 2007283480
(71) Applicant: NATIONAL INSTITUTE OF AGROBIOLOGICAL SCIENCES, Tsukuba-shi Ibaraki 305-8602 (JP)
(72) Inventor: MIYAO, Akio, Tsukuba-shi Ibaraki 305-8602 (JP)
(74) Representative: HOFFMANN EITLE
(86) International application number: PCT/JP2008/069897
(87) International publication number: WO 2009/057757

(57) **Abstract**

It is intended to provide a base sequence determination program, a base sequence determination device, and a base sequence determination method capable of constructing the whole genome sequence from an enormous amount of short base sequences of approximately several tens-base-long without referring to existing base sequences. In the invention, base sequences of two kinds of parent lineages carrying a genetic mutation and base sequences of a plurality of descending progenies of the generation after segregation are each analyzed, and segregation at a polymorphic site is referred to as an index of connection of the base sequences, and fragments of the base sequences are connected while verifying the validity of the segregation.

SEQUENCE LISTING

<110> NATIONAL INSTITUTE OF AGROBIOLOGICAL SCIENCES

<120> Base sequence determining program, base sequence determining apparatus,
and base sequence determining method

<130> PA-19671

<140> EP 08844737.0
<141> 2008 10 31

<150> JP 2007-0283480
<151> 2007 10 31

<160> 107

<170> PatentIn version 3.1

<210> 1
<211> 30
<212> DNA
<213> Oryza sativa

<220>
<223> DNA Sequence 1

<400> 1
taaaccctaa accctaaacc ctaaacccta 30

<210> 2
<211> 30
<212> DNA
<213> Oryza sativa

<220>
<223> DNA Sequence 2

<400> 2
taactcaaga gagacagaat ggaagataga 30

<210> 3
<211> 30
<212> DNA
<213> Oryza sativa

<220>
<223> DNA Sequence 3

<400> 3
   actgaaactt atactttaat attttttgag 30

<210> 4
<211> 30
<212> DNA
<213> Oryza sativa

<220>
<223> DNA Sequence 4

<400> 4
gcacgtcgag gaatgcgcga gccgacaacg 30

<210> 5

<211>  30
<212>  DNA
<213>  Oryza sativa

<220>
<223>  DNA Sequence 5

<400>  5
cacgtcgagg aatgcgcgag ccgacaacgc                                        30


<210>  6
<211>  30
<212>  DNA
<213>  Oryza sativa

<220>
<223>  DNA Sequence 6

<400>  6
acgtcgagga atgcgcgagc cgacaacgcc                                        30


<210>  7
<211>  30
<212>  DNA
<213>  Oryza sativa

<220>
<223>  DNA Sequence 7

<400>  7
cgtcgaggaa tgcgcgagcc gacaacgccg                                        30


<210>  8
<211>  30
<212>  DNA
<213>  Oryza sativa

<220>
<223>  DNA Sequence 8

<400>  8
gtcgaggaat gcgcgagccg acaacgccgc                                        30


<210>  9
<211>  30
<212>  DNA
<213>  Oryza sativa

<220>
<223>  DNA Sequence 9

<400>  9
tcgaggaatg cgcgagccga caacgccgcc                                        30


<210>  10
<211>  30
<212>  DNA
<213>  Oryza sativa

<220>
<223>  DNA Sequence 10

<400>  10

cgaggaatgc gcgagccgac aacgccgccg                                    30


<210>  11
<211>  30
<212>  DNA
<213>  Oryza sativa

<220>
<223>  DNA Sequence 11

<400>  11
gtccgcgctc gggctccttc acctgctcga                                    30


<210>  12
<211>  30
<212>  DNA
<213>  Oryza sativa

<220>
<223>  DNA Sequence 12

<400>  12
gtccgcgctc gggctccttc acctgctcga                                    30


<210>  13
<211>  30
<212>  DNA
<213>  Oryza sativa

<220>
<223>  DNA Sequence 13

<400>  13
tccgcgctcg ggctccttca cctgctcgat                                    30


<210>  14
<211>  30
<212>  DNA
<213>  Oryza sativa

<220>
<223>  DNA Sequence 14

<400>  14
ccgcgctcgg gctccttcac ctgctcgatg                                    30


<210>  15
<211>  30
<212>  DNA
<213>  Oryza sativa

<220>
<223>  DNA Sequence 15

<400>  15
cgcgctcggg ctccttcacc tgctcgatgc                                    30


<210>  16
<211>  30
<212>  DNA
<213>  Oryza sativa

<220>
<223>  DNA Sequence 16

<400>  16
gcgctcgggc tccttcacct gctcgatgcc                                        30


<210>  17
<211>  29
<212>  DNA
<213>  Oryza sativa

<220>
<223>  DNA Sequence 17

<400>  17
gctcgggctc cttcacctgc tcgatgccg                                         29


<210>  18
<211>  30
<212>  DNA
<213>  Oryza sativa

<220>
<223>  DNA Sequence 18

<400>  18
ctcgggctcc ttcacctgct cgatgccgac                                        30


<210>  19
<211>  30
<212>  DNA
<213>  Oryza sativa

<220>
<223>  DNA Sequence 19

<400>  19
tcgggctcct tcacctgctc gatgccgacg                                        30


<210>  20
<211>  30
<212>  DNA
<213>  Oryza sativa

<220>
<223>  DNA Sequence 20

<400>  20
gtccgcgctc gggctccttc acctgctcgg                                        30


<210>  21
<211>  30
<212>  DNA
<213>  Oryza sativa

<220>
<223>  DNA Sequence 21

<400>  21
gtccgcgctc gggctccttc acctgctcgg                                        30


<210>  22

<211>  30
<212>  DNA
<213>  Oryza sativa

<220>
<223>  DNA Sequence 22

<400>  22
tccgcgctcg ggctccttca cctgctcggc                                        30


<210>  23
<211>  30
<212>  DNA
<213>  Oryza sativa

<220>
<223>  DNA Sequence 23

<400>  23
ccgcgctcgg gctccttcac ctgctcggcg                                        30


<210>  24
<211>  30
<212>  DNA
<213>  Oryza sativa

<220>
<223>  DNA Sequence 24

<400>  24
cgcgctcggg ctccttcacc tgctcggcgg                                        30


<210>  25
<211>  30
<212>  DNA
<213>  Oryza sativa

<220>
<223>  DNA Sequence 25

<400>  25
gcgctcgggc tccttcacct gctcggcggc                                        30


<210>  26
<211>  30
<212>  DNA
<213>  Oryza sativa

<220>
<223>  DNA Sequence 26

<400>  26
cgctcgggct ccttcacctg ctcggcggcg                                        30


<210>  27
<211>  30
<212>  DNA
<213>  Oryza sativa

<220>
<223>  DNA Sequence 27

<400>  27

gctcgggctc cttcacctgc tcggcggcgc                                    30

<210>  28
<211>  30
<212>  DNA
<213>  Oryza sativa

<220>
<223>  DNA Sequence 28

<400>  28
ctcgggctcc ttcacctgct cggcggcgcc                                    30

<210>  29
<211>  30
<212>  DNA
<213>  Oryza sativa

<220>
<223>  DNA Sequence 29

<400>  29
tcgggctcct tcacctgctc ggcggcgccg                                    30

<210>  30
<211>  30
<212>  DNA
<213>  Oryza sativa

<220>
<223>  DNA Sequence 30

<400>  30
gcacgtcgag gaatgcgcga gccgacaacg                                    30

<210>  31
<211>  30
<212>  DNA
<213>  Oryza sativa

<220>
<223>  DNA Sequence 31

<400>  31
gcacgtcgag gaatgcgcga gccgacaacg                                    30

<210>  32
<211>  30
<212>  DNA
<213>  Oryza sativa

<220>
<223>  DNA Sequence 32

<400>  32
gcacgtcgag gaatgcgcga gccgacaaca                                    30

<210>  33
<211>  30
<212>  DNA
<213>  Oryza sativa

<220>
<223>  DNA Sequence 33

<400>  33
gcacgtcgag gaatgcgcga gccgacaaca                                        30


<210>  34
<211>  30
<212>  DNA
<213>  Oryza sativa

<220>
<223>  DNA Sequence 34

<400>  34
gcacgtcgag gaatgcgcga gccgacaacg                                        30


<210>  35
<211>  30
<212>  DNA
<213>  Oryza sativa

<220>
<223>  DNA Sequence 35

<400>  35
gcacgtcgag gaatgcgcga gccgacaaca                                        30


<210>  36
<211>  30
<212>  DNA
<213>  Oryza sativa

<220>
<223>  DNA Sequence 36

<400>  36
gcacgtcgag gaatgcgcga gccgacaacg                                        30


<210>  37
<211>  30
<212>  DNA
<213>  Oryza sativa

<220>
<223>  DNA Sequence 37

<400>  37
gcacgtcgag gaatgcgcga gccgacaacg                                        30


<210>  38
<211>  30
<212>  DNA
<213>  Oryza sativa

<220>
<223>  DNA Sequence 38

<400>  38
gcacgtcgag gaatgcgcga gccgacaaca                                        30


<210>  39

<211>  30
<212>  DNA
<213>  Oryza sativa

<220>
<223>  DNA Sequence 39

<400>  39
gcacgtcgag gaatgcgcga gccgacaaca                                        30


<210>  40
<211>  30
<212>  DNA
<213>  Oryza sativa

<220>
<223>  DNA Sequence 40

<400>  40
gcacgtcgag gaatgcgcga gccgacaacg                                        30


<210>  41
<211>  30
<212>  DNA
<213>  Oryza sativa

<220>
<223>  DNA Sequence 41

<400>  41
gcacgtcgag gaatgcgcga gccgacaaca                                        30


<210>  42
<211>  30
<212>  DNA
<213>  Oryza sativa

<220>
<223>  DNA Sequence 42

<400>  42
ttcggggtgg acacgggcga catgaacgag                                        30


<210>  43
<211>  30
<212>  DNA
<213>  Oryza sativa

<220>
<223>  DNA Sequence 43

<400>  43
ttcggggtgg acacgggcga catgaacgag                                        30


<210>  44
<211>  30
<212>  DNA
<213>  Oryza sativa

<220>
<223>  DNA Sequence 44

<400>  44

ttcggggtgg acacgggcga catgaacgaa                                    30

<210>  45
<211>  30
<212>  DNA
<213>  Oryza sativa

<220>
<223>  DNA Sequence 45

<400>  45
ttcggggtgg acacgggcga catgaacgaa                                    30

<210>  46
<211>  30
<212>  DNA
<213>  Oryza sativa

<220>
<223>  DNA Sequence 46

<400>  46
ttcggggtgg acacgggcga catgaacgag                                    30

<210>  47
<211>  30
<212>  DNA
<213>  Oryza sativa

<220>
<223>  DNA Sequence 47

<400>  47
ttcggggtgg acacgggcga catgaacgaa                                    30

<210>  48
<211>  30
<212>  DNA
<213>  Oryza sativa

<220>
<223>  DNA Sequence 48

<400>  48
ttcggggtgg acacgggcga catgaacgag                                    30

<210>  49
<211>  30
<212>  DNA
<213>  Oryza sativa

<220>
<223>  DNA Sequence 49

<400>  49
ttcggggtgg acacgggcga catgaacgag                                    30

<210>  50
<211>  30
<212>  DNA
<213>  Oryza sativa

<220>
<223>  DNA Sequence 50

<400>  50
ttcggggtgg acacgggcga catgaacgaa                                        30


<210>  51
<211>  30
<212>  DNA
<213>  Oryza sativa

<220>
<223>  DNA Sequence 51

<400>  51
ttcggggtgg acacgggcga catgaacgaa                                        30


<210>  52
<211>  30
<212>  DNA
<213>  Oryza sativa

<220>
<223>  DNA Sequence 52

<400>  52
ttcggggtgg acacgggcga catgaacgag                                        30


<210>  53
<211>  30
<212>  DNA
<213>  Oryza sativa

<220>
<223>  DNA Sequence 53

<400>  53
ttcggggtgg acacgggcga catgaacgaa                                        30


<210>  54
<211>  30
<212>  DNA
<213>  Oryza sativa

<220>
<223>  DNA Sequence 54

<400>  54
cggcgttcgt gatggtgtac gcagaggagg                                        30


<210>  55
<211>  30
<212>  DNA
<213>  Oryza sativa

<220>
<223>  DNA Sequence 55

<400>  55
cggcgttcgt gatggtgtac gcagaggagg                                        30


<210>  56

<211>  30
<212>  DNA
<213>  Oryza sativa

<220>
<223>  DNA Sequence 56

<400>  56
cggcgttcgt gatggtgtac gcagaggaga                                        30


<210>  57
<211>  30
<212>  DNA
<213>  Oryza sativa

<220>
<223>  DNA Sequence 57

<400>  57
cggcgttcgt gatggtgtac gcagaggaga                                        30


<210>  58
<211>  30
<212>  DNA
<213>  Oryza sativa

<220>
<223>  DNA Sequence 58

<400>  58
cggcgttcgt gatggtgtac gcagaggagg                                        30


<210>  59
<211>  30
<212>  DNA
<213>  Oryza sativa

<220>
<223>  DNA Sequence 59

<400>  59
cggcgttcgt gatggtgtac gcagaggagg                                        30


<210>  60
<211>  30
<212>  DNA
<213>  Oryza sativa

<220>
<223>  DNA Sequence 60

<400>  60
cggcgttcgt gatggtgtac gcagaggagg                                        30


<210>  61
<211>  30
<212>  DNA
<213>  Oryza sativa

<220>
<223>  DNA Sequence 61

<400>  61

cggcgttcgt gatggtgtac gcagaggaga                                        30

<210>  62
<211>  30
<212>  DNA
<213>  Oryza sativa

<220>
<223>  DNA Sequence 62

<400>  62
cggcgttcgt gatggtgtac gcagaggagg                                        30

<210>  63
<211>  30
<212>  DNA
<213>  Oryza sativa

<220>
<223>  DNA Sequence 63

<400>  63
cggcgttcgt gatggtgtac gcagaggaga                                        30

<210>  64
<211>  30
<212>  DNA
<213>  Oryza sativa

<220>
<223>  DNA Sequence 64

<400>  64
cggcgttcgt gatggtgtac gcagaggaga                                        30

<210>  65
<211>  30
<212>  DNA
<213>  Oryza sativa

<220>
<223>  DNA Sequence 65

<400>  65
cggcgttcgt gatggtgtac gcagaggaga                                        30

<210>  66
<211>  30
<212>  DNA
<213>  Oryza sativa

<220>
<223>  DNA Sequence 66

<400>  66
gcacgtcgag gaatgcgcga gccgacaacg                                        30

<210>  67
<211>  30
<212>  DNA
<213>  Oryza sativa

<220>
<223>  DNA Sequence 67

<400>  67
gtccgcgctc gggctccttc acctgctcga                                        30


<210>  68
<211>  30
<212>  DNA
<213>  Oryza sativa

<220>
<223>  DNA Sequence 68

<400>  68
ttcggggtgg acacgggcga catgaacgag                                        30


<210>  69
<211>  30
<212>  DNA
<213>  Oryza sativa

<220>
<223>  DNA Sequence 69

<400>  69
ttcggggtgg acacgggcga catgaacgag                                        30


<210>  70
<211>  30
<212>  DNA
<213>  Oryza sativa

<220>
<223>  DNA Sequence 70

<400>  70
gtccgcgctc gggctccttc acctgctcgg                                        30


<210>  71
<211>  30
<212>  DNA
<213>  Oryza sativa

<220>
<223>  DNA Sequence 71

<400>  71
cggcgttcgt gatggtgtac gcagaggagg                                        30


<210>  72
<211>  199
<212>  DNA
<213>  Oryza sativa

<220>
<223>  DNA Sequence 72

<400>  72
gcacgtcgag gaatgcgcga gccgacaacg ccgccgtggg gagcacttcc catcgtggtg      60

cccttcctag accccaagtc ctaggcggcg gcctcctgcg tctgcacctc ctggagcgcc     120

gccttcggcg ccgacggtct ctgggtgcag ctctgccgct cctagttccc gtccgcgctc    180

gggctccttc acctgctcg                                                  199


<210>  73
<211>  30
<212>  DNA
<213>  Oryza sativa

<220>
<223>  DNA Sequence 73

<400>  73
tctgggtgca gctctgccgc tcctagttcc                                       30


<210>  74
<211>  30
<212>  DNA
<213>  Oryza sativa

<220>
<223>  DNA Sequence 74

<400>  74
ctgggtgcag ctctgccgct cctagttccc                                       30


<210>  75
<211>  30
<212>  DNA
<213>  Oryza sativa

<220>
<223>  DNA Sequence 75

<400>  75
tgggtgcagc tctgccgctc ctagttcccg                                       30


<210>  76
<211>  30
<212>  DNA
<213>  Oryza sativa

<220>
<223>  DNA Sequence 76

<400>  76
gggtgcagct ctgccgctcc tagttcccgt                                       30


<210>  77
<211>  30
<212>  DNA
<213>  Oryza sativa

<220>
<223>  DNA Sequence 77

<400>  77
ggtgcagctc tgccgctcct agttcccgtc                                       30


<210>  78
<211>  30
<212>  DNA

<213>  Oryza sativa

<220>
<223>  DNA Sequence 78

<400>  78
gtgcagctct gccgctccta gttcccgtcc                                        30


<210>  79
<211>  30
<212>  DNA
<213>  Oryza sativa

<220>
<223>  DNA Sequence 79

<400>  79
tgcagctctg ccgctcctag ttcccgtccg                                        30


<210>  80
<211>  30
<212>  DNA
<213>  Oryza sativa

<220>
<223>  DNA Sequence 80

<400>  80
gcagctctgc cgctcctagt tcccgtccgc                                        30


<210>  81
<211>  30
<212>  DNA
<213>  Oryza sativa

<220>
<223>  DNA Sequence 81

<400>  81
cagctctgcc gctcctagtt cccgtccgcg                                        30


<210>  82
<211>  30
<212>  DNA
<213>  Oryza sativa

<220>
<223>  DNA Sequence 82

<400>  82
agctctgccg ctcctagttc ccgtccgcgc                                        30


<210>  83
<211>  30
<212>  DNA
<213>  Oryza sativa

<220>
<223>  DNA Sequence 83

<400>  83
gctctgccgc tcctagttcc cgtccgcgct                                        30

<210>  84
<211>  30
<212>  DNA
<213>  Oryza sativa

<220>
<223>  DNA Sequence 84

<400>  84
ctctgccgct cctagttccc gtccgcgctc                                        30


<210>  85
<211>  30
<212>  DNA
<213>  Oryza sativa

<220>
<223>  DNA Sequence 85

<400>  85
tctgccgctc ctagttcccg tccgcgctcg                                        30


<210>  86
<211>  30
<212>  DNA
<213>  Oryza sativa

<220>
<223>  DNA Sequence 86

<400>  86
ctgccgctcc tagttcccgt ccgcgctcgg                                        30


<210>  87
<211>  30
<212>  DNA
<213>  Oryza sativa

<220>
<223>  DNA Sequence 87

<400>  87
tgccgctcct agttcccgtc cgcgctcggg                                        30


<210>  88
<211>  30
<212>  DNA
<213>  Oryza sativa

<220>
<223>  DNA Sequence 88

<400>  88
gccgctccta gttcccgtcc gcgctcgggc                                        30


<210>  89
<211>  30
<212>  DNA
<213>  Oryza sativa

<220>
<223>  DNA Sequence 89

<400>  89
ccgctcctag ttcccgtccg cgctcgggct                                        30


<210>  90
<211>  30
<212>  DNA
<213>  Oryza sativa

<220>
<223>  DNA Sequence 90

<400>  90
cgctcctagt tcccgtccgc gctcgggctc                                        30


<210>  91
<211>  30
<212>  DNA
<213>  Oryza sativa

<220>
<223>  DNA Sequence 91

<400>  91
gctcctagtt cccgtccgcg ctcgggctcc                                        30


<210>  92
<211>  30
<212>  DNA
<213>  Oryza sativa

<220>
<223>  DNA Sequence 92

<400>  92
ctcctagttc ccgtccgcgc tcgggctcct                                        30


<210>  93
<211>  30
<212>  DNA
<213>  Oryza sativa

<220>
<223>  DNA Sequence 93

<400>  93
tcctagttcc cgtccgcgct cgggctcctt                                        30


<210>  94
<211>  30
<212>  DNA
<213>  Oryza sativa

<220>
<223>  DNA Sequence 94

<400>  94
cctagttccc gtccgcgctc gggctccttc                                        30


<210>  95
<211>  30
<212>  DNA

<213>  Oryza sativa

<220>
<223>  DNA Sequence 95

<400>  95
ctagttcccg tccgcgctcg ggctccttca                                        30


<210>  96
<211>  30
<212>  DNA
<213>  Oryza sativa

<220>
<223>  DNA Sequence 96

<400>  96
tagttcccgt ccgcgctcgg gctccttcac                                        30


<210>  97
<211>  30
<212>  DNA
<213>  Oryza sativa

<220>
<223>  DNA Sequence 97

<400>  97
agttcccgtc cgcgctcggg ctccttcacc                                        30


<210>  98
<211>  30
<212>  DNA
<213>  Oryza sativa

<220>
<223>  DNA Sequence 98

<400>  98
gttcccgtcc gcgctcgggc tccttcacct                                        30


<210>  99
<211>  30
<212>  DNA
<213>  Oryza sativa

<220>
<223>  DNA Sequence 99

<400>  99
ttcccgtccg cgctcgggct ccttcacctg                                        30


<210>  100
<211>  30
<212>  DNA
<213>  Oryza sativa

<220>
<223>  DNA Sequence 100

<400>  100
tcccgtccgc gctcgggctc cttcacctgc                                        30

<210>  101
<211>  30
<212>  DNA
<213>  Oryza sativa

<220>
<223>  DNA Sequence 101

<400>  101
cccgtccgcg ctcgggctcc ttcacctgct                                        30


<210>  102
<211>  30
<212>  DNA
<213>  Oryza sativa

<220>
<223>  DNA Sequence 102

<400>  102
ccgtccgcgc tcgggctcct tcacctgctc                                        30


<210>  103
<211>  30
<212>  DNA
<213>  Oryza sativa

<220>
<223>  DNA Sequence 103

<400>  103
cgtccgcgct cgggctcctt cacctgctcg                                        30


<210>  104
<211>  200
<212>  DNA
<213>  Oryza sativa

<220>
<223>  DNA Sequence 104

<400>  104
gcacgtcgag gaatgcgcga gccgacaacg ccgccgtggg gagcacttcc catcgtggtg      60

cccttcctag accccaagtc ctaggcggcg gcctcctgcg tctgcacctc ctggagcgcc     120

gccttcggcg ccgacggtct ctgggtgcag ctctgccgct cctagttccc gtccgcgctc     180

gggctccttc acctgctcga                                                 200


<210>  105
<211>  200
<212>  DNA
<213>  Oryza sativa

<220>
<223>  DNA Sequence 105

<400>  105
gcacgtcgag gaatgcgcga gccgacaacg ccgccgtggg gagcacttcc catcgtggtg      60

cccttcctag accccaagtc ctaggcggcg gcctcctgcg tctgcacctc ctggagcgcc     120

gccttcggcg ccgacggtct ctgggtgcag ctctgccgct cctagttccc gtccgcgctc 180

gggctccttc acctgctcgg 200

<210> 106
<211> 29
<212> DNA
<213> Oryza sativa

<220>
<223> DNA Sequence 106

<400> 106
cacgtcgagg aatgcgcgag ccgacaacg 29

<210> 107
<211> 29
<212> DNA
<213> Oryza sativa

<220>
<223> DNA Sequence 107

<400> 107
gtccgcgctc gggctccttc acctgctcg 29

## Description

### TECHNICAL FIELD

The present invention relates to a base sequence determination program determining the whole genome sequence, a base sequence determination device, and a base sequence determination method.

### BACKGROUND ART

In recent years, whole genome of a large number of organisms including humans has been sequenced. A base sequence determination device (sequencer) is used for the sequencing. At present, a base sequence determination device can sequence approximately 500 to 1500 bp per sample upon an analysis of a sample. Sequencing of the whole genome is completed by analyzing a tremendous number of samples, and connecting the base sequences thus obtained by overlapping identical segments (homologous segments) of individual base sequence data. Generally, a program such as Phred/Phrap (Nonpatent Literature 1), Cap 3 (Nonpatent Literature 2), and Arachne (Nonpatent Literature 3) is used for detection of segments to be overlapped based on sequences having approximately 500 to 1500 bp obtained by a base sequence determination device and for connection of the sequences. Also, there is a BLAST-like alignment tool called BLAT (Nonpatent Literature 4). BLAT enables accelerated processing by breaking the genome into nonoverlapping K-mers and placing an index on a random access memory (RAM).

Phred/Phrap has become virtually a standard program, which calculates overlapping of base sequences by the Smith-Waterman algorithm and outputs base sequences that are connected in consideration of quality data of each base. Cap 3 outputs more accurate base sequences by connecting base sequences while eliminating uncertain segments present in the terminal regions in individual base sequences. However, both Phred/Phrap and Cap 3 cannot distinguish completely identical, repetitive sequences from one another. On the other hand, Arachne analyzes a base sequence of each sample from both ends, and connects the base sequences by adding the analytical information obtained thereby. Therefore, even when repetitive sequences are present, Arachne is relatively capable of accurately connecting base sequences.

Nonpatent Literature 1: Ewing B, Green P., "Base-calling of automated sequencer traces using phred. II. Error probabilities.", Genome Res., 8(3), 186-194, 1998.
Nonpatent Literature 2: Huang X, Madan A.,"CAP3: A DNA sequence assembly program.", Genome Res., 9(9), 868-877, 1999.
Nonpatent Literature 3: Batzoglou S et al., "ARACHNE: a whole-genome shotgun assembler.", Genome Res., 12(1), 177-189, 2002.
Nonpatent Literature 4: W. James Kent, "BLAT - The BLAST-Like Alignment Tool", Genome Res., 12, 656-664, 2002.

### DISCLOSURE OF INVENTION

### PROBLEM TO BE SOLVED BY THE INVENTION

However, these programs are suitable for analyzing a BAC (bacterial artificial chromosome) clone that is approximately 100 kbp-long obtained by dividing the genome (in other words, connecting base sequences obtained by fragmenting a clone of approximately 100 kbp-long), but there is a problem that when an attempt is made to sequence the whole genome at once, these programs are incapable of correctly connecting one base sequence to another due to repetitive sequences scattering throughout the genome. Namely, a problem has been posed with these programs that there is a difficulty in reconstructing the whole genome sequence by connecting base sequences obtained by fragmenting the whole genome at once due to repetitive sequences present in the genome.

The present invention has been made in consideration of the above problem, and its object is to provide a base sequence determination program, a base sequence determination device, and a base sequence determination method capable of constructing the whole genome sequence from an enormous amount of short base sequences of approximately several tens-base-long without referring to existing base sequences.

### MEANS FOR SOLVING PROBLEM

A computer program product having a computer readable medium according to one aspect of the present invention includes programmed instructions for a base sequence determination method, wherein the instructions, when executed by an information processor including a control unit, and a storage unit, wherein the storage unit includes a database that stores a plurality of base sequences derived from each of a plurality of parents and a plurality of descendants thereof having a genetic polymorphism, cause the information processor to perform a step of connecting the base sequences by referring to the database included in the storage unit, using the genetic polymorphism as a marker, wherein the step is executed by the control unit.

A computer program product having a computer readable medium according to another aspect of the present invention includes programmed instructions for a base sequence determination method, wherein the instructions, when executed by an information processor including a control unit, and a storage unit, wherein the storage unit includes a database that stores a plurality of base sequences derived from each of a plurality of parents and a plurality of descendants thereof having a genetic polymorphism, cause the information processor to perform a searching step of searching, based on a target base sequence, which is the base sequence of the target parent, the base sequence to be connected to the target base sequence from the database included in the storage unit, a detecting step of detecting the genetic polymorphism between the parents based on the base sequence searched at the searching step, a verifying step of verifying whether a base sequence obtained from the identical parent is present in plural numbers in the base sequences searched at the searching step, an examining step of examining, when the genetic polymorphism is detected at the detecting step and it is not verified at the verifying step that a base sequence obtained from the identical parent is present in plural numbers, a genotype of the descendants at a site at which the genetic polymorphism is detected based on the base sequence of the descendants searched at the searching step, an assessing step of assessing whether the genotype of the descendants examined at the examining step and the genotype of the descendants that is previously examined match, and an extending step of extending, when the genotypes are assessed to match each other at the assessing step, the target base sequence based on the base sequence of the parent searched at the searching step, and, when it is verified at the verifying step that a base sequence obtained from the identical parent is present in plural numbers, extends the target base sequences separately with respect to each of the base sequences of the identical parent searched at the searching step, wherein the steps are executed by the control unit.

The computer program product according to still another aspect of the present invention, wherein the database is a relational database in which the base sequence of the individual and an individual-identifying information to uniquely distinguish the individual from another are stored in association with each other, is characterized by the searching step includes searching the base sequence using SQL, which is a database language.

The computer program product according to still another aspect of the present invention, wherein the storage unit further includes an index that can be used for a prefix search, produced with respect to the base sequence stored in the database, is characterized by the searching step includes conducting the prefix search for the base sequence by referring to the index.

A base sequence determination device according to still another aspect of the present invention includes a storage unit including a database that stores a plurality of base sequences derived from each of a plurality of parents and a plurality of descendants thereof having a genetic polymorphism, and a control unit connecting the base sequences by referring to the database included in the storage unit, using the genetic polymorphism as a marker.

A base sequence determination device according to still another aspect of the present invention includes a control unit, and a storage unit, wherein the storage unit includes a database that stores a plurality of base sequences derived from each of a plurality of parents and a plurality of descendants thereof having a genetic polymorphism, and the control unit includes a searching unit that searches, based on a target base sequence, which is the base sequence of the target parent, the base sequence to be connected to the target base sequence from the database included in the storage unit, a detecting unit that detects the genetic polymorphism between the parents based on the base sequence searched by the searching unit, a verifying unit that verifies whether a base sequence obtained from the identical parent is present in plural numbers in the base sequences searched by the searching unit, an examining unit that examines, when the genetic polymorphism is detected by the detecting unit and it is not verified by the verifying unit that a base sequence obtained from the identical parent is present in plural numbers, a genotype of the descendants at a site at which the genetic polymorphism is detected based on the base sequence of the descendants searched by the searching unit, an assessing unit that assesses whether the genotype of the descendants examined by the examining unit and the genotype of the descendants that is previously examined match, and an extending unit that extends, when the genotypes are assessed to match each other by the assessing unit, the target base sequence based on the base sequence of the parent searched by the searching unit, and, when it is verified by the verifying unit that a base sequence obtained from the identical parent is present in plural numbers, extends the target base sequences separately with respect to each of the base sequences of the identical parent searched by the searching unit.

The base sequence determination device according to still another aspect of the present invention includes the database is a relational database in which the base sequence of the individual and an individual-identifying information to uniquely distinguish the individual from another are stored in association with each other, and the searching unit searches the base sequence using SQL, which is a database language.

The base sequence determination device according to still another aspect of the present invention includes the storage unit further includes an index that can be used for a prefix search, produced with respect to the base sequence stored in the database, and the searching unit conducts the prefix search for the base sequence by referring to the index.

A base sequence determination method according to still another aspect of the present invention is executed by an information processor includes a control unit, and a storage unit, wherein the storage unit includes a database that stores a plurality of base sequences derived from each of a plurality of parents and a plurality of descendants thereof having a genetic polymorphism, the method including a step of connecting the base sequences by referring to the database included in the storage unit, using the genetic polymorphism as a marker, wherein the step is executed by the control unit.

A base sequence determination method according to still another aspect of the present invention is executed by an information processor including a control unit, and a storage unit, wherein the storage unit includes a database that stores a plurality of base sequences derived from each of a plurality of parents and a plurality of descendants thereof having a genetic polymorphism, the method comprising a searching step of searching, based on a target base sequence, which is the base sequence of the target parent, the base sequence to be connected to the target base sequence from the database included in the storage unit, a detecting step of detecting the genetic polymorphism between the parents based on the base sequence searched at the searching step, a verifying step of verifying whether a base sequence obtained from the identical parent is present in plural numbers in the base sequences searched at the searching step, an examining step of examining, when the genetic polymorphism is detected at the detecting step and it is not verified at the verifying step that a base sequence obtained from the identical parent is present in plural numbers, a genotype of the descendants at a site at which the genetic polymorphism is detected based on the base sequence of the descendants searched at the searching step, an assessing step of assessing whether the genotype of the descendants examined at the examining step and the genotype of the descendants that is previously examined match, and an extending step of extending, when the genotypes are assessed to match each other at the assessing step, the target base sequence based on the base sequence of the parent searched at the searching step, and, when it is verified at the verifying step that a base sequence obtained from the identical parent is present in plural numbers, extends the target base sequences separately with respect to each of the base sequences of the identical parent searched at the searching step, wherein the steps are executed by the control unit.

The base sequence determination method according to still another aspect of the present invention, wherein the database is a relational database in which the base sequence of the individual and an individual-identifying information to uniquely distinguish the individual from another are stored in association with each other, includes the searching step includes searching the base sequence using SQL, which is a database language.

The base sequence determination method according to still another aspect of the present invention, wherein the storage unit further includes an index that can be used for a prefix search, produced with respect to the base sequence stored in the database, includes the searching step includes conducting the prefix search for the base sequence by referring to the index.

### Effect of the Invention

The invention connects a plurality of base sequences by referring to the database that stores a plurality of base sequences derived from each of a plurality of parents and a plurality of descendants thereof having a genetic polymorphism, using the genetic polymorphism as a marker. Specifically, the invention, (1) searches, based on a target base sequence, which is the base sequence of the target parent, the base sequence to be connected to the target base sequence from the database that stores a plurality of base sequences derived from each of a plurality of parents and a plurality of descendants thereof having a genetic polymorphism, (2) detects the genetic polymorphism between the parents based on the base sequence searched, (3) verifies whether a base sequence obtained from the identical parent is present in plural numbers in the base sequences searched, (4) examines, when the genetic polymorphism is detected and it is not verified that a base sequence obtained from the identical parent is present in plural numbers, a genotype of the descendants at a site at which the genetic polymorphism is detected based on the base sequence of the descendants searched, (5) assesses whether the genotype of the descendants examined and the genotype of the descendants that is previously examined match, and (6) extends, when the genotypes are assessed to match each other, the target base sequence based on the base sequence of the parent searched, and, when it is verified that a base sequence obtained from the identical parent is present in plural numbers, extends the target base sequences separately with respect to each of the base sequences of the identical parent searched.

According to the above, an effect is obtained that the whole genome sequence can be constructed from an enormous amount of short base sequences of approximately several tens-base-long without referring to existing base sequences. In other words, an enormous amount of short base sequences of approximately several tens-base-long obtained by fragmenting the whole genome at once can be correctly connected, including repetitive sequences scattering throughout the genome. As a result, an effect is obtained that the whole genome can be sequenced at once. Namely, an effect is obtained that the whole genome sequence can be reconstructed by connecting base sequences obtained by fragmenting the whole genome at once. Further, an effect is obtained that not only the whole genome sequence but also the information on genetic polymorphism in each descendant can be simultaneously obtained.

### BRIEF DESCRIPTION OF DRAWINGS

[FIG. 1] FIG. 1 is a principle configuration diagram showing a basic principle of the present invention.
[FIG. 2] FIG. 2 is a principle configuration diagram showing a basic principle of the present invention.
[FIG. 3] FIG. 3 is a principle configuration diagram showing a basic principle of the present invention.
[FIG. 4] FIG. 4 is a block diagram showing a configuration of the base sequence determination device 100.
[FIG. 5] FIG. 5 is a diagram for showing an example of information stored in a data table 106a1 that is a component of a base sequence database 106a.
[FIG. 6] FIG. 6 is a flow chart for showing an example of a database building process executed by a control unit 102 of the base sequence determination device 100.
[FIG. 7] FIG. 7 is a flow chart for showing an example of a base sequence determination process executed by the control unit 102 of the base sequence determination device 100.
[FIG. 8] FIG. 8 is a diagram showing an example of an output made in the base sequence determination process.
[FIG. 9] FIG. 9 is a diagram showing an example of an output made in the base sequence determination process.
[FIG. 10] FIG. 10 is a flow chart for showing an example of an output result analyzing process executed by the control unit 102 of the base sequence determination device 100.

### EXPLANATIONS OF LETTERS OR NUMERALS

- 100: base sequence determination device
- 102: control unit
- 102a: searching unit
- 102b: extending unit
- 102c: detecting unit
- 102d: verifying unit
- 102e: examining unit
- 102f: assessing unit
- 104: communication interface
- 106: storage unit
- 106a: base sequence database
- 106a1: data table
- 106a2: index file
- 106b: target base sequence file
- 106c: genotype file
- 106d: correct base sequence file
- 108: input/output interface
- 110: input device
- 112: output device
- 300: network

### BEST MODE(S) FOR CARRYING OUT THE INVENTION

The following describes an embodiment of a base sequence determination program, a base sequence determination device, and a base sequence determination method according to the present invention in detail with reference to the drawings. The embodiment is illustrative only, and is not intended to limit the present invention in any way.

### [1. Overview of the Invention]

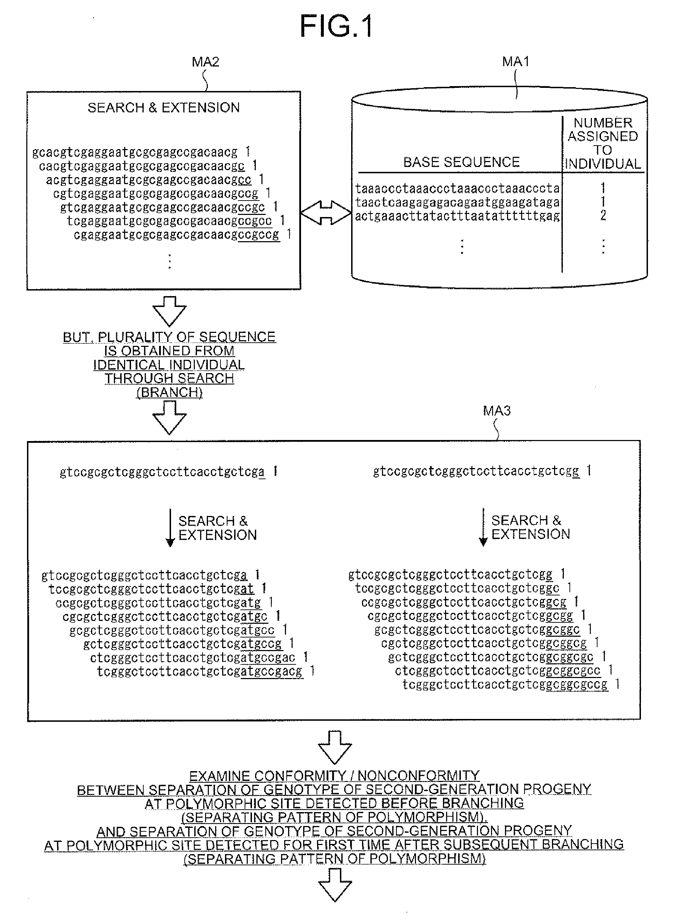

The following outlines the present invention with reference to FIGs. 1 to 3. FIGs. 1 to 3 are principle configuration diagrams showing a basic principle of the present invention.

In the invention, genomic DNA derived from two kinds of parents having a genetic polymorphism (hereinafter, it may be simply referred to as "polymorphism") and genomic DNA derived from a plurality of descendants of the parents are obtained in advance. Specifically, base sequences of a parent 1 and a parent 2 as well as base sequences of the second-generation progenies of the parents (F2) 3 to 6 are obtained, and the base sequences thus obtained are summarized in a Table indicated in MA1 in FIG. 1 (a Table in which a base sequence and a number assigned to an individual are associated with each other: a name of the Table is "seq", a name of the column is "seq", and a number assigned to an individual is "no"). In the present explanation, it is supposed that "each base sequence is 30-base-long and all the base sequence data have been obtained with respect to each individual with overlap by one base" for the sake of convenience.

In the invention, basically the following [operation 1] to [operation 3] are repeatedly carried out. By doing so, as depicted in MA2 in FIG. 1, a randomly-selected base sequence of the parent 1 "gcacgtcgaggaatgogcaagccgacaacg" is extended at its 3' side by one base at a time.
[Operation 1] A base sequence of the parent 1 is set as a target base sequence (as a default, a 30-base-long sequence, "gcacgtcgaggaatgcgcgagccgacaacg", which is randomly selected from among base sequences of the parent 1 in Table indicated in MA1 in FIG. 1, is set), and from the Table indicated in MA1 in FIG. 1, a base following the base at the 3' side of the target base sequence is searched by a prefix search using a SQL sentence "SELECT seq, no FROM seq WHERE seq LIKE' cacgtcgaggaatgcgcgagccgacaacg%';", which is a database language. When the genome of the parent 2 is sequenced, a base sequence derived from the parent 2 is searched using a base sequence of the parent 2 as a target base sequence.
[Operation 2] When a sequence of the parent 1 "cacgtogaggaatgcgcgagccgacaacgc 1" is returned as a search result, it is determined that a base following the base at the 3' side of the target base sequence is "c".
[Operation 3] The determined base "c" is connected to the 3' side of the target base sequence "gcacgtcgaggaatgcgcgagccgacaacg", thereby the target base sequence is extended.

However, while carrying out the operations, when, for example, a base sequence of the parent 1 "gtccgcgctcgggctccttcacctgctcga 1" and another base sequence of the parent 1 "gtccgcgctcgggctccttcacctgctcgg 1" are obtained as a result of a search in [Operation 1], whether a base following the base at the 3' side of the target base sequence is "a" or "g" cannot be figured out only by the base sequences obtained through the search. Specifically, when a plurality of 30-base-long sequences that prefix-matches a 29-base-long query sequence "gtccgcgctcgggctccttcacctgctcg" is obtained from the genome of an identical individual (the parent 1) through the search, namely, when a plurality of base sequences (plural kinds of base sequences) that are each present in different locations in the genome of an identical individual (the parent 1) is simultaneously detected, it cannot be figured out which base sequence should be selected from among the base sequences obtained through the search. Supposing that extension is continued by the operations for each base sequence obtained through the search, the base sequence would be extended to have completely different base sequence from one another as depicted in MA3 in FIG. 1 ("atgccgacg" and "gcggcgccg").

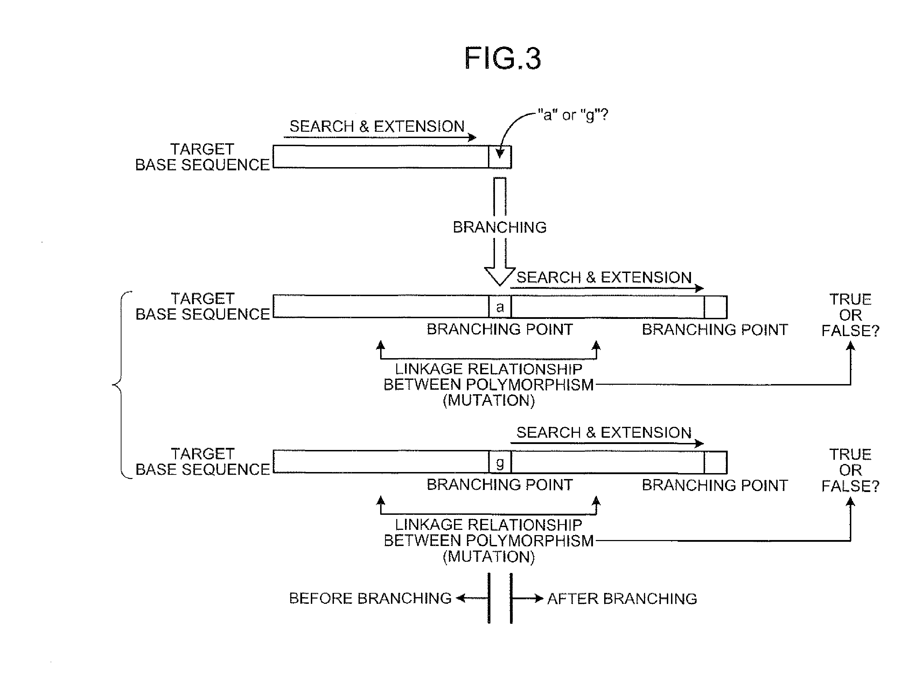

In the invention, to judge whether a base following the base at the 3' side of the target base sequence is "a" or "g", as depicted in FIG. 3, a target base sequence to which "a" is connected and a target base sequence to which "g" is connected are branched from each other, then the operations are carried out, whereby the target base sequences are each separately extended, then, while the extension is continued, segregation of polymorphism before and after the branching, namely a linkage relationship between mutations is examined, and based on the examination, validity of each connection is verified. In other words, in the invention, a target base sequence to which "a" is connected and a target base sequence to which "g" is connected are branched from each other, then the operations are carried out, whereby the target base sequences are each separately extended, then, while extension is continued, when a polymorphism is detected for the first time after branching with respect to each of the target base sequences, a difference between the polymorphism detected for the first time (polymorphism after branching) and an original polymorphism (polymorphism before branching) is examined, and based on the examination, validity of each connection is verified. When no polymorphism is found from one branching to the next branching, branching is repeated until a polymorphism is found, and a branching in which conformity is found in a linkage relationship of mutation is selected from among all combinations of branching.

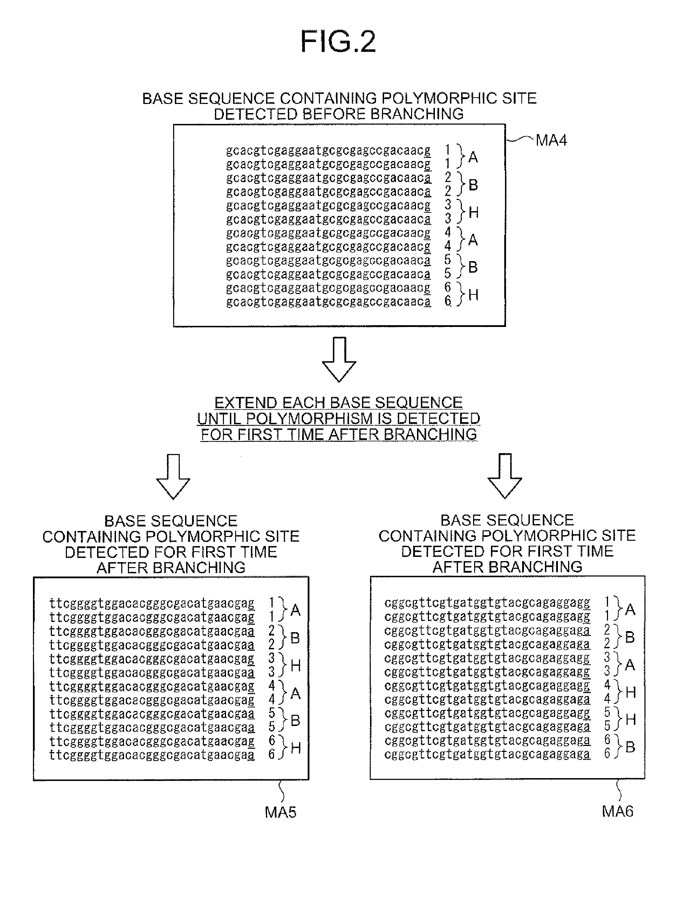

For example, it is supposed that base sequences of the parent 1 and the parent 2, containing a polymorphic site detected before branching as well as base sequences of the second-generation progenies 3 to 6 corresponding to the polymorphic site are as depicted in MA4 in FIG. 2. In the above case, each base at the 3' end of the parent 1 is "g" and "g" (which is provided as parent type 1 (type A)), and each base at the 3' end of the parent 2 is "a" and "a" (which is provided as parent type 2 (type B)). Also, each base at the 3' end of the second-generation progeny 3 is "g" and "a", each base at the 3' end of the second-generation progeny 4 is "g" and "g", each base at the 3' end of the second-generation progeny 5 is "a" and "a", and each base at the 3' end of the second-generation progeny 6 is "g" and "a." That is, a genotype of the second-generation progeny 3 is a heterozygous (type H), a genotype of the second-generation progeny 4 is type A, a genotype of the second-generation progeny 5 is type B, and a genotype of the second-generation progeny 6 is type H. From the above, a genotype of a site at which a polymorphism is detected can be expressed as "ABHABA."

It is supposed that, as a result that each target base sequence is extended after branching, and then a polymorphism is detected for the first time, base sequences of the parent 1 and the parent 2, containing the polymorphic site, as well as base sequences of the second-generation progenies 3 to 6 corresponding to the polymorphic site are each found to be as depicted in MA5 and MA6 in FIG. 2, respectively. Then, a genotype of each individual at the polymorphic site is examined with respect also to each of the base sequences depicted in MA5 and MA6 in FIG. 2 in a similar fashion to before branching. As a result, a genotype of the base sequences indicated in MA5 in FIG. 2 can be expressed as "ABHABH", and a genotype of the base sequences indicated in MA6 in FIG. 2 can be expressed as "ABAHHB." The base sequence indicated in MA5 in FIG. 2 are such that "atgccgacg" follows after "···tgctcg" as depicted in the left side of MA3 in FIG. 1, which is a result of continuing extension until a polymorphism is detected for the first time after branching. Also, the base sequence indicated in MA6 in FIG. 2 are such that "gcggcgccg" follows after "···tgctcg" as depicted in the right side of MA3 in FIG. 1, which is a result of continuing extension until a polymorphism is detected for the first time after branching.

In the invention, the genotype before branching "ABHABH" and the genotype after branching are compared, and a target base sequence in which the genotypes match each other is judged as a correct one to be extended. That is, in the invention, a genotype at a polymorphic site before branching and a genotype at a polymorphic site after subsequent branching are compared. According to the present explanation, it is judged that extending by adding "ataccgacg" successively after "···tgctcg" as depicted in the left side of MA3 in FIG. 1 is correct.

As explained above, in the invention, base sequences of two kinds of parent lineages having a polymorphism and a base sequence of a descendant (for example, a second-generation progeny (F2)) are compiled in a database, and short base sequences are connected using the polymorphism as an index. In the invention, base sequences of two kinds of parent lineages carrying a genetic mutation and base sequences of the descendants of the generation after segregation are each analyzed, and segregation of a polymorphic site is referred to as an index of connection of the base sequences, and fragments of the base sequences are connected while verifying the validity of the segregation. In the invention, base sequences are assembled while verifying a linkage relationship between mutations by simultaneously analyzing base sequences of the descendants.

According to the above, distinction of multiple identical base sequences present in the genome is enabled, and as a result, an assembly with a high accuracy can be realized. The whole genome sequence can be constructed from an enormous amount of short base sequences of approximately several tens-base-long without referring to existing base sequences. In other words, an enormous amount of short base sequences of approximately several tens-base-long obtained by fragmenting the whole genome at once can be correctly connected, including repetitive sequences scattering throughout the genome. As a result, the whole genome can be sequenced at once. Namely, the whole genome sequence can be reconstructed by connecting base sequences obtained by fragmenting the whole genome at once. Further, not only the whole genome sequence but also the information on genetic polymorphism in each descendant can be simultaneously obtained.

A sequencer capable of sequencing approximately 30 bases per sample by analyzing several tens of millions of samples at once has been developed presently, but, when short base sequences are connected, discrimination of a correct sequence is generally difficult at repetitive sequence segments that are present in abundance in the genome. So far, an effort has been made to avoid incorrect connection by making a length of a fragment that can be sequenced in one analysis longer.

However, according to the invention, the length per fragment does not matter, and even when the fragments obtained from a sequencer are as short as approximately 30-base-long (for example, 30- to 70-base-long), as long as the sufficient amount of data are secured so that overlaps are attained for sure, a correct sequence can be distinguished using a genetic mutation as an index, even when homologous segments are scattered in a plurality of chromosomes. Therefore, according to the invention, repetitive sequence segments can also be connected correctly, and as a result, the whole genome sequence can be reconstructed by connecting a large amount of relatively short base sequences.

Further, according to the invention, a large number of base sequences as short as approximately 30-base-long are stored in a relational database, and an index that can be used for a prefix search such as a B-tree type index is produced with respect to the base sequences stored in the database, and then the base sequences are extended by repeating a prefix search using a SQL sentence. By doing so a candidate base sequence to be connected successively can be searched fast, and as a result, assembling of the whole genomic DNA can be realized accurately at high speed. That is, according to the invention, base sequences can be connected accurately at high speed.

According to the invention, a mutation can be scanned in advance, genotypes of all of the 30 bases including the scanned mutation can be determined, and a base sequence can be extended by the operations. By doing so assembling of base sequences can be further efficiently realized.

It is supposed in the present explanation that the sufficient amounts of data to cover the whole genome with an accuracy of one base are obtained, but, according to the invention, even when such an amount of data that the genome is covered approximately by several-fold with respect to each individual is obtained, a partial base sequence of approximately 17-base-long can be obtained by shifting by one base at a time from the 5' end of a target base sequence, and the partial base sequence thus obtained can be set as a query sequence, and then the target base sequence can be extended while producing an alignment of base sequences that are returned in response to the query. When a base that is different from one individual to another (mismatch) is detected in the case when a 30-base-long sequence data is searched by a prefix search using a 17- to 29-base-long query sequence derived from the parent 1, and an alignment of base sequences that have matched data derived from the parents as well as the second-generation progeny is produced, segregation of the detected polymorphic base between individuals can be used as an index for the judgment.

According to the invention, a genomic sequence of any kind of organisms that can mate can be sequenced with higher efficiency than ever before, and all the information on polymorphism that is simultaneously mapped can also be obtained. According to the invention, segments that cannot be cloned can also be connected with a high accuracy. When detailed data on segregation of phenotypes in an analytical group are obtained, identification of a causative gene for QTL (Quantitative Trait Locus) is possible in principle according to the invention. The genomic sequences of various kinds of organisms that are determined by the invention have immeasurable industrial utility value.

### [2. Configuration of the System]

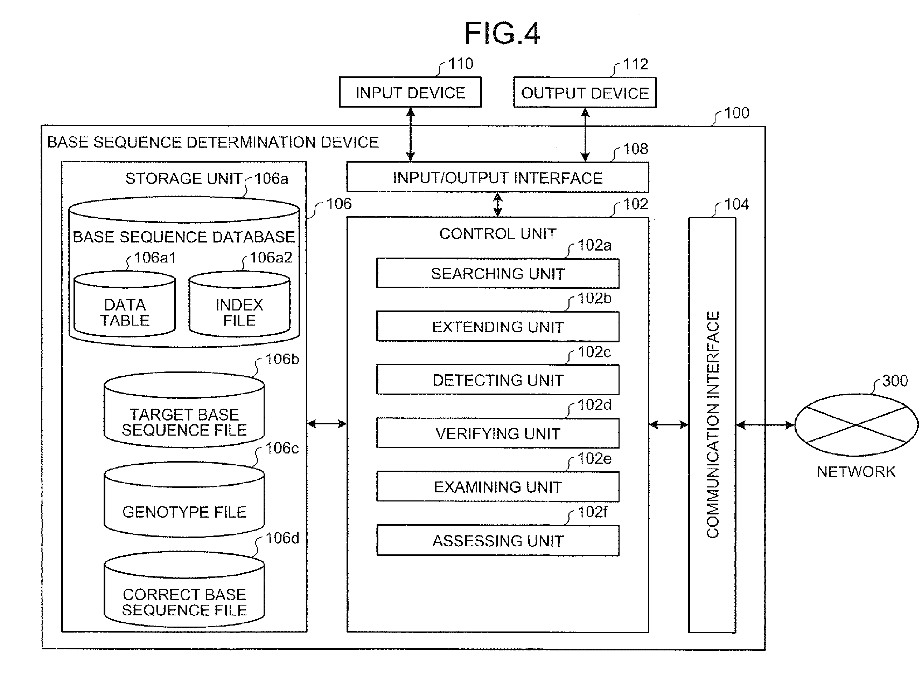

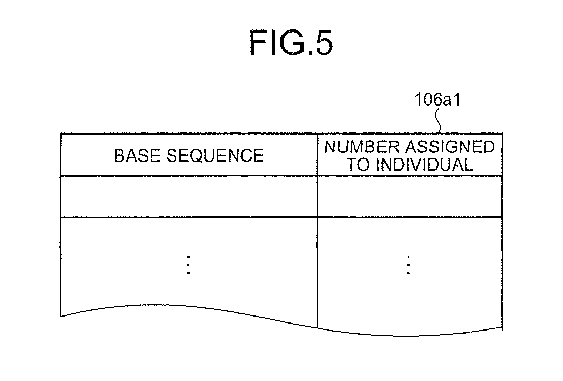

The following describes a configuration of the base sequence determination device 100 with reference to FIGs. 4 and 5. FIG. 4 is a block diagram showing a configuration of the base sequence determination device 100 to which the present embodiment is applied, and conceptually shows only parts related to the present invention.

The base sequence determination device 100 is provided with a control unit 102 such as a central processing unit (CPU) that integrally controls the entire base sequence determination device 100, a communication interface 104 which communicatably connects the base sequence determination device 100 to a network 300 via a communication device such as a router, and a wire or wireless communication line such as an exclusive line, a storage unit 106 that stores various databases, tables, and files, and an input/output interface 108 connected to an input device 110 and an output device 112, and the units are communicatably connected through an optional communication channel.

The storage unit 106 is a storage unit such as a memory device that is RAM, read only memory (ROM) or the like, a fixed disk device like hard disk (HD), a fixed disk device, an optical disk, and the like. The storage unit 106 stores a base sequence database 106a, a target base sequence file 106b, a genotype file 106c, and a correct base sequence file 106d as shown.

The base sequence database 106a stores a plurality of base sequences derived from each of a plurality of parents and a plurality of descendants thereof (for example, second-generation progenies of the parents) having a genetic polymorphism, and an index that is produced with respect to the base sequences (for example, a B-tree index). The base sequence database 106a consists of a data table 106a1 and an index file 106a2. The following describes information stored in the data table 106a1 that is a component of the base sequence database 106a with reference to FIG. 5. FIG. 5 is a diagram for showing an example of information stored in the data table 106a1 that is a component of the base sequence database 106a. As shown in FIG. 5, the data table 106a1 is a relational database in which a base sequence of an individual and a number assigned to an individual to uniquely distinguish the individual from another are stored in association with each other.

Referring back to FIG. 4, the index file 106a2, which is a constitutive element of the base sequence database 106a, stores an index that is produced with respect to the base sequences stored in the data table 106a1 and that can be used for a prefix search (for example, a B-tree index). A target base sequence file 106b stores a target base sequence, which is a base sequence of a parent that is subjected to extension. A default target base sequence is randomly set from among base sequences of any of parents whose genome is wished to be sequenced. The genotype file 106c stores a genotype of each individual that has been examined in an examining unit 102e described below. The correct base sequence file 106d stores a correct base sequence, which is finally determined as a correct base sequence after continuation of extension of the target base sequence.

The communication interface 104 mediates communication between the base sequence determination device 100 and the network 300 (the communication device such as a router). That is, the communication interface 104 has a function of transmitting data to other terminals through a communication line.

The input/output interface 108 connects to the input device 110 and the output device 112. The output device 112 corresponds to a display (monitor), a speaker, a printer, and the like. The input device 110 corresponds to a keyboard, a mouse, a microphone, and a monitor that has pointing device function with the mouse.

The control unit 102 has an internal memory that stores a control program such as an operating system (OS), a program defining various procedures, and required data, and performs information processing for executing various processing by the programs or the like. The control unit 102 is provided with a searching unit 102a, an extending unit 102b, a detecting unit 102c, a verifying unit 102d, an examining unit 102e, and an assessing unit 102f as the figure.

The searching unit 102a searches, based on a target base sequence, which is the base sequence of the target parent, the base sequence to be connected to the target base sequence from the data table 106a1 in the base sequence database 106a included in the storage unit 106. The searching unit 102a searches the base sequence using SQL, which is a database language by referring to the index file 106a2 in the base sequence database 106a included in the storage unit 106.

The extending unit 102b extends the target base sequence based on the base sequence of the parent searched by the searching unit 102a. When the genotypes are assessed to match each other by the assessing unit 102f to be described later, the extending unit 102b extends the target base sequence based on the base sequence of the parent searched by the searching unit 102a. When it is verified by the verifying unit 102d that a base sequence obtained from the identical parent is present in plural numbers to be described later, the extending unit 102b extends the target base sequences separately with respect to each of the base sequences of the identical parent searched by the searching unit 102a.

The detecting unit 102c detects the genetic polymorphism between the parents based on the base sequence searched by the searching unit 102a. The verifying unit 102d verifies whether a base sequence obtained from the identical parent is present in plural numbers in the base sequences searched by the searching unit 102a. The examining unit 102e examines, when the genetic polymorphism is detected by the detecting unit 102c and it is not verified by the verifying unit 102d that a base sequence obtained from the identical parent is present in plural numbers, a genotype of each individual (a parent, and a descendant) at a site at which the genetic polymorphism is detected based on the base sequence of each individual (a parent, and a descendant) searched by the searching unit 102a. The assessing unit 102f assesses whether the genotype of each individual (any one of a parent and a descendant or both) examined by the examining unit 102e and the genotype of each individual (any one of a parent and a descendant or both) that is previously examined match.

The network 300 has function of connecting the base sequence determination device 100 with the external system, and may at least include internet, intranet, LAN (wired/wireless), VAN, personal computer communication network, public telephone network (analog/digital), leased circuit (analog/digital), cable TV network, mobile phone switching network/ packet-switching data network by IMT2000, GSM, PDC/PDC-P, or the like, radio paging network, local wireless network such as Bluetooth (R), PHS network, satellite communication network such as CS, BS, and ISDB, and the like. The base sequence determination device 100 can transmit and receive various data through any wired or wireless network.

### [3. Processing of the System]

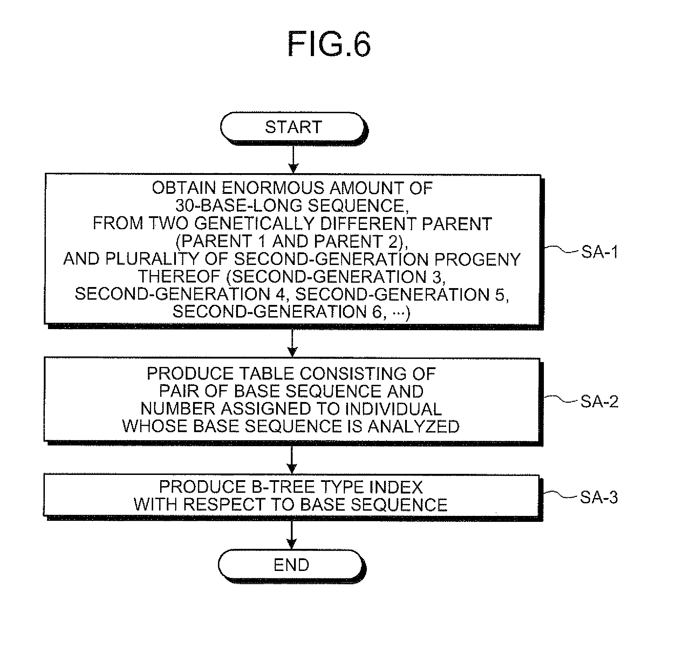

The following describes some processing executed by the control unit 102 of the base sequence determination device 100 with reference to FIGs. 6 to 10.

### [3-1. Database Building Process]

The following first describes a database building process executed by the control unit 102 of the base sequence determination device 100 with reference to FIG. 6. FIG. 6 is a flow chart for showing an example of the database building process executed by the control unit 102 of the base sequence determination device 100.

The control unit 102 obtains an enormous amount (several tens of thousands to several hundred millions) of 30-base-long sequences, which are obtained by analyzing two genetically different parents (parent 1 and parent 2) and a plurality of the second-generation progenies thereof (second-generation 3, second-generation 4, second-generation 5, second-generation 6, ···) with a sequencer (step SA-1).

The control unit 102 produces a table consisting of pairs of the base sequences obtained at step SA-1 and numbers assigned to individuals to distinguish the individual whose base sequence is analyzed, and stores the table thus produced in the data table 106a1 in the base sequence database 106a (step SA-2).

The control unit 102 produces a B-tree type index with respect to the base sequences obtained at step SA-1, and stores the B-tree type index thus produced in the index file 106a2 in the base sequence database 106a (step SA-3).

### [3-2. Base Sequence Determination Process]

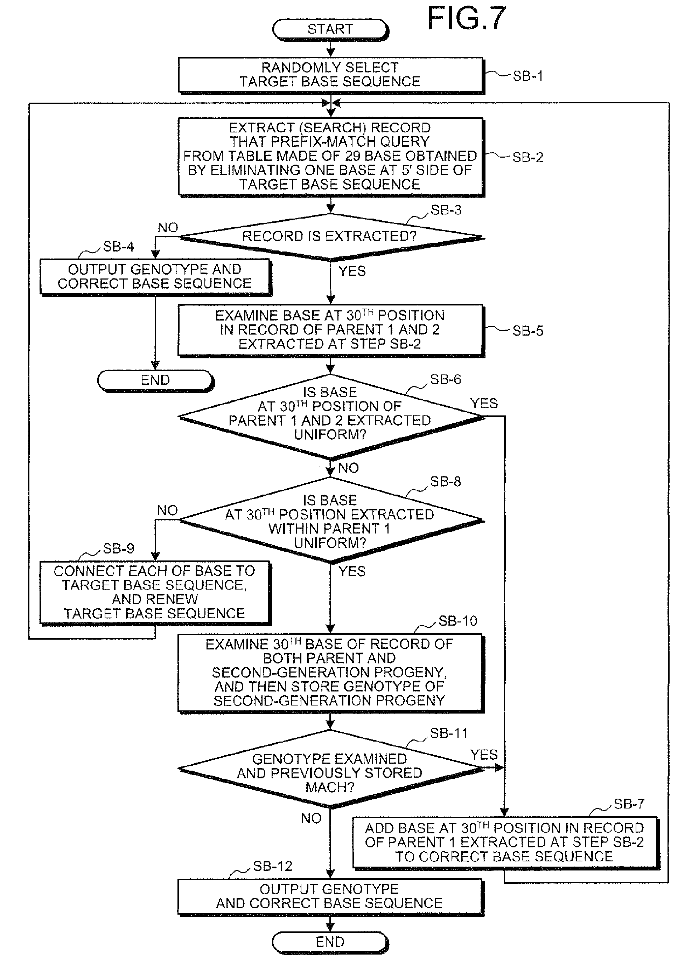

The following describes a base sequence determination process executed by the control unit 102 of the base sequence determination device 100 with reference to FIGs. 7 to 9. FIG. 7 is a flow chart for showing an example of a base sequence determination process executed by the control unit 102 of the base sequence determination device 100.

The control unit 102 randomly selects a 30-base-long target sequence from among the base sequences of the parent 1 that are stored in the data table 106a1 in the base sequence database 106a, and stores a copy of the target base sequence thus selected in the correct base sequence file 106d as a correct base sequence (step SB-1).

Then, in the searching unit 102a, the control unit 102 sets 29 bases obtained by eliminating one base at the 5' side of the target base sequence determined at step SB-1 (29 bases from the 3' side of the target base sequence) as a query, and then extracts (searches) a record that prefix-matches the query set as above using a SQL sentence from the data table 106a1 in the base sequence database 106a by referring to the B-tree type index stored in the index file 106a2 (step SB-2).

When no record is extracted at step SB-2 (step SB-3: No), the control unit 102 outputs a genotype stored in the genotype file 106c and a correct base sequence stored in the correct base sequence file 106d to the output device 112 (step SB-4), thereby terminating the above process.

When a record is extracted at step SB-2 (step SB-3: Yes), the control unit 102 examines bases at the 30^{th} position from the 5' sides of the records of the parents 1 and 2 extracted at step SB-2 (the first base at the 3' side) in the detecting unit 102c (step SB-5).

When the bases of the parents 1 and 2 that are examined at step SB-5 are detected as uniform, namely, detected as non-polymorphic (step SB-6: Yes), the control unit 102 adds the base of the parent 1 examined as above to the 3' side of the target base sequence and to the 3' side of the correct base sequence stored in the correct base sequence file 106d (step SB-7) in the extending unit 102b, and then the process is returned to step SB-2.

When the bases of the parents 1 and 2 that are examined at step SB-5 are verified to be non-uniform (step SB-6: No), and further, the bases are verified to be non-uniform within the parent 1 in the verifying unit 102d, namely, a plurality of records of the parent 1 that differ from one another at the 30^{th} base (a plurality of kinds of records of the parent 1) are obtained through a search (step SB-8: No), the control unit 102 produces such a number of copies of the target base sequences that is equal to the number of the bases examined and adds (connects) each of the bases thus examined to the 3' side of each of the target base sequences thus produced in the extending unit 102b, whereby a plurality of target base sequences are renewed (step SB-9). The process is then returned to SB-2.

Then, when the bases of the parents 1 and 2 that are examined at step SB-5 are verified to be non-uniform (step SB-6: No), and further, the bases are verified to be uniform within the parent 1 in the verifying unit 102d (step SB-8: Yes), namely, detected as polymorphic, the control unit 102 examines the 30^{th} bases from the 5' sides (the bases at the 3' ends) of the base sequences of parents (parent 1 and parent 2) and the second-generation progenies (second-generation 3, second-generation 4, second-generation 5, second-generation 6, ···) that are obtained through a search at step SB-2 in the examining unit 102e, and examines a genotype of each second-generation progeny based on each of the bases examined as above to store the genotypes thus examined in the genotype file 106c (step SB-10).

When the genotypes examined at step SB-10 and the genotypes previously stored in the genotype file 106c are assessed to match in the assessing unit 102f in the control unit 102 (step SB-11: Yes), the process is returned to step SB-7.

When the genotypes examined at step SB-10 and the genotypes previously stored in the genotype file 106c are not assessed to match in the assessing unit 102f in the control unit 102 (step SB-11: No), the control unit 102 outputs the genotypes stored in the genotype file 106c and the correct base sequences up to before branching that are stored in the correct base sequence file 106d to the output device 112 (step SB-12), thereby terminating the above process.

As explained above, a correct base sequence is obtained from among a plurality of branching points that are generated by extension in the present process. Namely, in the present process, at the branching point, target base sequences having each of the bases extracted at the 3' ends thereof are stored with information on polymorphism in separate files. Each target base sequence is extended using 29 bases at the 3' side of the target base sequence stored in each file as a query. Then, when a polymorphism observed as the extension is continued does not match the original polymorphism, the file being used is deleted, and a similar process is newly conducted on a next file. On the other hand, when a polymorphism observed as the extension is continued matches the original polymorphism, all the files except the file containing the matched base sequence are deleted, and then, extension is further continued using the remaining file.

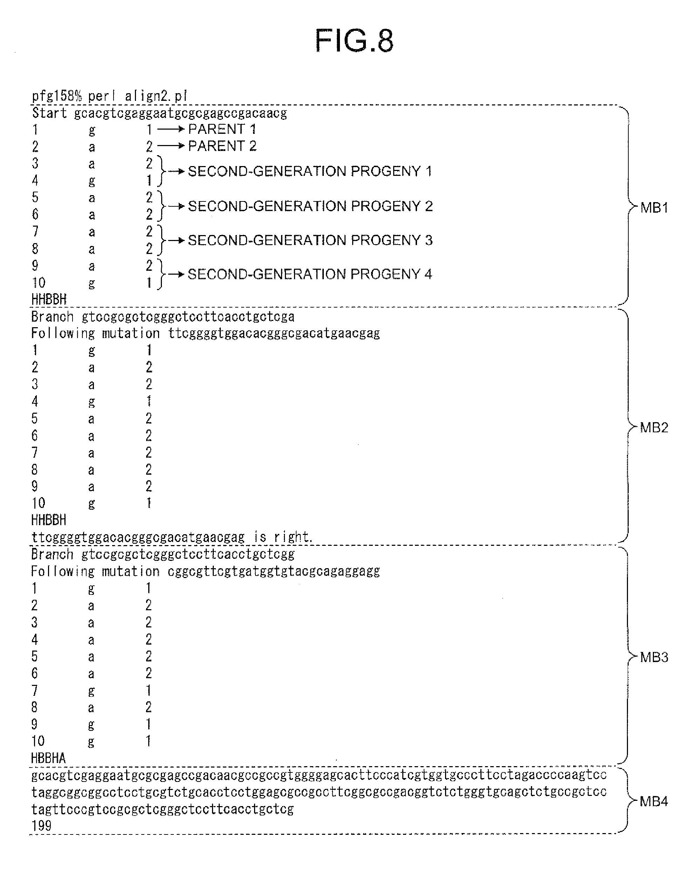

In the present process, when a 30^{th} base from the 5' side of the base sequence of the parent 1 (Start "gcacgtcgaggaatgcgcgagccgacaacg") is polymorphic, namely, when a 30^{th} base differs between the parents (for example, the 30^{th} base derived from the parent 1(A) is "g (=1)" while the 30^{th} base derived from the parent 2 (B) is "a (=2)") as depicted in MB1 in FIG. 8, polymorphisms and genotypes of second-generation progenies are examined. As a result of examination, when the 30^{th} bases derived from the second-generation progeny 1 are "a" and "g" and the genotype is heterozygous (type H); the 30^{th} bases derived from the second-generation progeny 2 are "a" and "a" and the genotype is parent type 2 (type B); the 30^{th} base derived from the second-generation progeny 3 are "a" and "a" and the genotype is parent type 2 (type B); and the 30^{th} base derived from the second-generation progeny 4 are "a" and "g" and the genotype is heterozygous (type H), the genotype of the parents and the second-generation progenies at this position is expressed as "HHBBH" (the leading "H" in this genotype represents a genotype of the parent, and subsequent "HBBH" represents genotypes of the second-generation progenies 1 to 4. In the present explanation, because polymorphisms of the parents 1 and 2 are determined together, the genotype of the parents will be always "H" at a polymorphic site). Because the example in FIG. 8 imitates diploid rice genome data, the number "1" represents data derived from the parent 1; the number "2" represents data derived from the parent 2; the numbers "3 and 4" represent data derived from the second-generation progeny 1; the numbers "5 and 6" represent data derived from the second-generation progeny 2, the numbers "7 and 8" represent data derived from the second-generation progeny 3; and the numbers "9 and 10" represent data derived from the second-generation progeny 4. Because a polymorphism is impossible to be segregated within each parent, in the example in FIG. 8, only the number "1" is assigned to the parent 1 and only the number "2" is assigned to the parent 2, instead of assigning the numbers "1 and 2" to the parent 1. In the above case, three kinds of combinations (i.e., only "a" is observed, only "g" is observed, or "a" and "g" are observed at almost the same frequency) can be obtained as actual data of the second-generation progenies. When only "a" is observed in a second-generation progeny, the progeny is expressed as the parent 1 (type A), when only "g" is observed in a second-generation progeny, the progeny is expressed as the parent 2 (type B), and when "a" and "g" are observed at almost the same frequency in a second-generation progeny, the progeny is expressed as the heterozygous (type H). In the present process, data derived from each of sister chromosomes in a second-generation progeny are separately input into database (for example, 3 and 4) for the sake of easier understanding.

In the present process, when two kinds of base sequences that differ at a 30^{th} base are obtained from the parent 1 through a search, setting the 30^{th} base as a branching point, a branching sequence "gtccgcgctcgggctccttcacctgctcga" as depicted in MB2 in FIG. 8 and a branching sequence "gtccgcgctcgggctccttcacctgctcgg" as depicted in MB3 in FIG. 8 are separately extended until a new polymorphism is detected between the parents 1 and 2. Then, in the present process, polymorphisms and genotypes of the second-generation progenies are again examined at a position at which a new polymorphism is detected between the parents 1 and 2 (the 30^{th} base "g" from the 5' side of the sequence "ttcggggtggacacgggcgacatgaacgag" depicted as Following mutation sequence in MB2 in FIG. 8 and the 30^{th} base "g" from the 5' side of the sequence "cggcgttcgtgatggtgtacgcagaggagg" depicted as Following mutation sequence in MB3 in FIG. 8). As a result of examination, for example, when the genotype of the parents and the second-generation progenies is expressed as "HHBBH" with respect to Following mutation sequence depicted in MB2 in FIG. 8, and the genotype of the parents and the second-generation progenies is expressed as "HBBHA" with respect to Following mutation sequence depicted in MB3 in FIG. 8, it is assessed that extending the target base sequence in accordance with Following mutation sequence depicted in MB2 in FIG. 8, which matches the previously-examined genotype "HHBBH", is correct in the present process. Then, in the present process, for example, a correct base sequence depicted in MB4 in FIG. 8 is finally output.

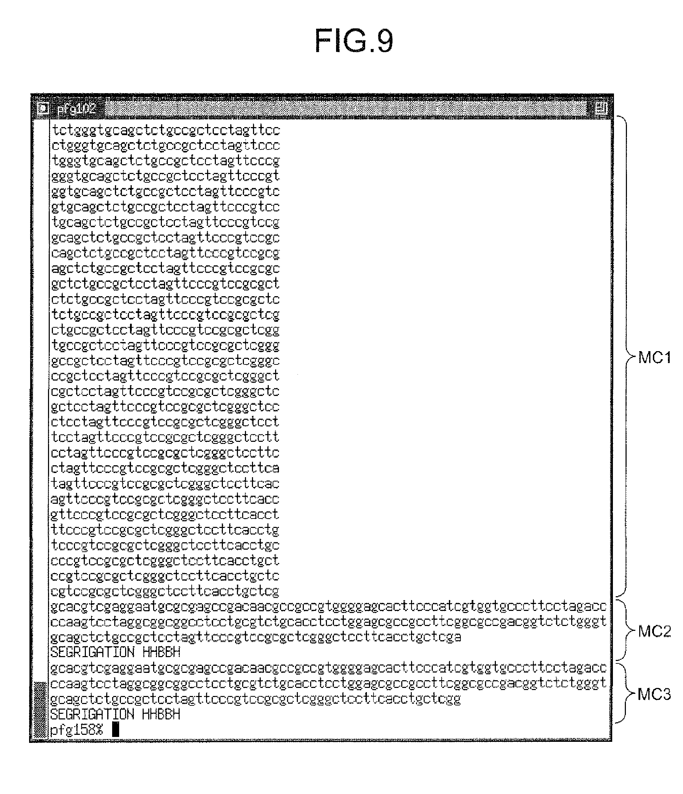

Further, in the present process, as depicted in MC1 in FIG. 9, using 29 bases at the 3' side of the base sequence derived from the parent 1 as depicted in the first line of MC1 in FIG. 9 as a query sequence, base sequences to be connected thereto are repeatedly searched by shifting the query sequence to the 3' side by one base at a time. Then, in the present process, when a plurality of kinds of different base sequences are obtained from the parent 1 through a search, each of such base sequences is separately output to the correct base sequence file 106d with the previously-examined genotype "HHBBH" with respect to a base that is obtained as the 3' end of the base sequence depicted in MC2 in FIG. 9 through a search and a base that is obtained as the 3' end of the base sequence depicted in MC3 in FIG. 9 through a search, and then, extension is continued with respect to each of the correct base sequence files. Whether a genotype of a polymorphic site subsequently detected matches "HHBBH" is examined with respect to each of the correct base sequence files, then, when they do not match, the correct base sequence file is deleted, or when they match, the correct base sequence file will be remained, and then, extension is continued with respect to the remaining file.

### [3-3. Output Result Analyzing Process]

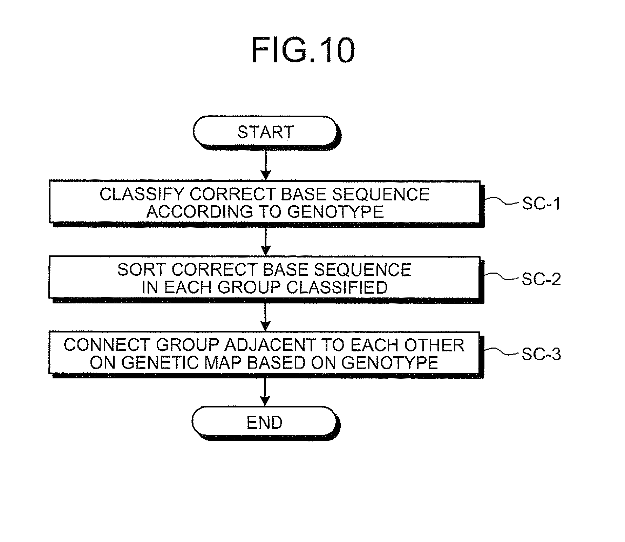

The following describes an output result analyzing process executed by the control unit 102 of the base sequence determination device 100 with reference to FIG. 10. FIG. 10 is a flow chart for showing an example of an output result analyzing process executed by the control unit 102 of the base sequence determination device 100.

Based on the correct base sequences and the genotypic information output by the base sequence determination process, the control unit 102 classifies the correct base sequences into a plurality of groups according to the genotype (step SC-1).

The control unit 102 sorts the correct base sequences in each group classified at step SC-1 (step SC-2).

The control unit 102 connects groups adjacent to each other on a genetic map within the groups classified at step SC-1 based on the genotype (step SC-3).

### [4. Summary of the present embodiments and other embodiments]

As explained above, the base sequence determination device 100 connects a plurality of base sequences by referring to the database that stores a plurality of base sequences derived from each of a plurality of parents and a plurality of descendants thereof having a genetic polymorphism, using the genetic polymorphism as a marker. Specifically, the invention, searches, based on a target base sequence, which is the base sequence of the target parent, the base sequence to be connected to the target base sequence from the database that stores a plurality of base sequences derived from each of a plurality of parents and a plurality of descendants thereof having a genetic polymorphism, detects the genetic polymorphism between the parents based on the base sequence searched, verifies whether a base sequence obtained from the identical parent is present in plural numbers in the base sequences searched, examines, when the genetic polymorphism is detected and it is not verified that a base sequence obtained from the identical parent is present in plural numbers, a genotype of the descendants at a site at which the genetic polymorphism is detected based on the base sequence of the descendants searched, assesses whether the genotype of the descendants examined and the genotype of the descendants that is previously examined match, and extends, when the genotypes are assessed to match each other, the target base sequence based on the base sequence of the parent searched, and, when it is verified that a base sequence obtained from the identical parent is present in plural numbers, extends the target base sequences separately with respect to each of the base sequences of the identical parent searched.

According to the above, the whole genome sequence can be constructed from an enormous amount of short base sequences of approximately several tens-base-long without referring to existing base sequences. In other words, an enormous amount of short base sequences of approximately several tens-base-long obtained by fragmenting the whole genome at once can be correctly connected, including repetitive sequences scattering throughout the genome. As a result, the whole genome can be sequenced at once. Namely, the whole genome sequence can be reconstructed by connecting base sequences obtained by fragmenting the whole genome at once. Further, not only the whole genome sequence but also the information on genetic polymorphism in each descendant can be simultaneously obtained.

According to the base sequence determination device 100, the database is a relational database in which the base sequence of the individual and an individual-identifying information to uniquely distinguish the individual from another are stored in association with each other, and the base sequence determination device 100 searches the base sequence using SQL. Therefore, a base sequence to be successively connected can be efficiently searched.

The base sequence determination device 100 further includes an B-tree type index that can be used for a prefix search, produced with respect to the base sequence stored in the database, and conducts the prefix search for the base sequence by referring to the B-tree type index. Therefore, a base sequence to be successively connected can be searched fast.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth. For example, in the embodiment, the base sequence determination device 100 performs various processes as a stand-alone device. However, the base sequence determination device 100 can be configured to perform processes in response to request from a client terminal, which is a separate unit, and return the process results to the client terminal. All the automatic processes explained in the present embodiment can be, entirely or partially, carried out manually. Similarly, all the manual processes explained in the present embodiment can be, entirely or partially, carried out automatically by a known method. The process procedures, the control procedures, specific names, information including registration data for each process and various parameters such as search conditions, display example, and database construction, mentioned in the description and drawings can be changed as required unless otherwise specified.

The constituent elements of the base sequence determination device 100 are merely conceptual and may not necessarily physically resemble the structures shown in the drawings. For instance, the device need not necessarily have the structure that is illustrated. For example, the process functions performed by each device of the base sequence determination device 100, specially the each process function performed by the control unit 102, can be entirely or partially realized by CPU and a computer program executed by the CPU or by a hardware using wired logic. The computer program, recorded on a recording medium to be described later, can be mechanically read by the base sequence determination device 100 as the situation demands. In other words, the storage unit 106 such as ROM or HD stores the computer program that can work in coordination with OS to issue commands to the CPU and cause the CPU to perform various processes. The computer program is first loaded to RAM, and forms a control unit 102 in collaboration with the CPU. Alternatively, the computer program can be stored in any application program server connected to the base sequence determination device 100 via the network 300, and can be fully or partially loaded as the situation demands.

The computer-readable recording medium on which the computer program can be stored may be a portable type such as flexible disk, magneto optic (MO) disk, ROM, erasable programmable ROM (EPROM), electrically erasable programmable ROM (EEPROM), compact disk-read-only memory (CD-ROM), digital versatile disk (DVD), or a communication medium that stores the computer program for a short term such as communication channels or carrier waves that transmit the computer program over the network 300 such as local area network (LAN), wide area network (WAN), and the Internet. Computer program refers to a data processing method written in any computer language and written method, and can have software codes and binary codes in any format. The computer program can be a dispersed form in the form of a plurality of modules or libraries, or can perform various functions in collaboration with a different program such as the OS. Any known configuration in the each device according to the embodiment can be used for reading the recording medium. Similarly, any known process procedure for reading or installing the computer program can be used.

The base sequence determination device 100 can also be realized by using any existing personal computer, workstation, and the like connected to peripheral equipments such as printer, monitor, and image scanner, and can be operated by executing software (that includes computer program, data, etc.) that implements the method according to the present invention in the personal computer or workstation.

The distribution and integration of the base sequence determination device 100 are not limited to those illustrated in the figures. The device as a whole or in parts can be functionally or physically distributed or integrated in an arbitrary unit according to various attachments or how the device is to be used. For example, each database may form a stand-alone database device, and a part of process may be executed by using common gateway interface (CGI).

### INDUSTRIAL APPLICABILITY

As described above, a base sequence determination program, a base sequence determination device, and a base sequence determination method related to the present invention have high applicability in various fields such as medical care, drag making, drug discovery, and biological investigation.

## Claims

1. A computer program product having a computer readable medium including programmed instructions for a base sequence determination method, wherein the instructions, when executed by an information processor including:
a control unit, and a storage unit, wherein
the storage unit includes:
a database that stores a plurality of base sequences derived from each of a plurality of parents and a plurality of descendants thereof having a genetic polymorphism,
cause the information processor to perform:
a step of connecting the base sequences by referring to the database included in the storage unit, using the genetic polymorphism as a marker,
wherein the step is executed by the control unit.

2. A computer program product having a computer readable medium including programmed instructions for a base sequence determination method, wherein the instructions, when executed by an information processor including:
a control unit, and a storage unit, wherein
the storage unit includes:
a database that stores a plurality of base sequences derived from each of a plurality of parents and a plurality of descendants thereof having a genetic polymorphism,
cause the information processor to perform:
a searching step of searching, based on a target base sequence, which is the base sequence of the target parent, the base sequence to be connected to the target base sequence from the database included in the storage unit;
a detecting step of detecting the genetic polymorphism between the parents based on the base sequence searched at the searching step;
a verifying step of verifying whether a base sequence obtained from the identical parent is present in plural numbers in the base sequences searched at the searching step;
an examining step of examining, when the genetic polymorphism is detected at the detecting step and it is not verified at the verifying step that a base sequence obtained from the identical parent is present in plural numbers, a genotype of the descendants at a site at which the genetic polymorphism is detected based on the base sequence of the descendants searched at the searching step;
an assessing step of assessing whether the genotype of the descendants examined at the examining step and the genotype of the descendants that is previously examined match; and
an extending step of extending, when the genotypes are assessed to match each other at the assessing step, the target base sequence based on the base sequence of the parent searched at the searching step, and, when it is verified at the verifying step that a base sequence obtained from the identical parent is present in plural numbers, extends the target base sequences separately with respect to each of the base sequences of the identical parent searched at the searching step,
wherein the steps are executed by the control unit.

3. The computer program product according to claim 10, wherein
the database is a relational database in which the base sequence of the individual and an individual-identifying information to uniquely distinguish the individual from another are stored in association with each other, and
the searching step includes searching the base sequence using SQL, which is a database language.

4. The computer program product according to claim 11, wherein
the storage unit further includes:
an index that can be used for a prefix search, produced with respect to the base sequence stored in the database, and
the searching step includes conducting the prefix search for the base sequence by referring to the index.

5. A base sequence determination device comprising:
a storage unit including a database that stores a plurality of base sequences derived from each of a plurality of parents and a plurality of descendants thereof having a genetic polymorphism; and
a control unit connecting the base sequences by referring to the database included in the storage unit, using the genetic polymorphism as a marker.

6. A base sequence determination device comprising:
a control unit, and a storage unit, wherein
the storage unit includes:
a database that stores a plurality of base sequences derived from each of a plurality of parents and a plurality of descendants thereof having a genetic polymorphism, and
the control unit includes:
a searching unit that searches, based on a target base sequence, which is the base sequence of the target parent, the base sequence to be connected to the target base sequence from the database included in the storage unit;
a detecting unit that detects the genetic polymorphism between the parents based on the base sequence searched by the searching unit;
a verifying unit that verifies whether a base sequence obtained from the identical parent is present in plural numbers in the base sequences searched by the searching unit;
an examining unit that examines, when the genetic polymorphism is detected by the detecting unit and it is not verified by the verifying unit that a base sequence obtained from the identical parent is present in plural numbers, a genotype of the descendants at a site at which the genetic polymorphism is detected based on the base sequence of the descendants searched by the searching unit;
an assessing unit that assesses whether the genotype of the descendants examined by the examining unit and the genotype of the descendants that is previously examined match; and
an extending unit that extends, when the genotypes are assessed to match each other by the assessing unit, the target base sequence based on the base sequence of the parent searched by the searching unit, and, when it is verified by the verifying unit that a base sequence obtained from the identical parent is present in plural numbers, extends the target base sequences separately with respect to each of the base sequences of the identical parent searched by the searching unit.

7. The base sequence determination device according to claim 6, wherein
the database is a relational database in which the base sequence of the individual and an individual-identifying information to uniquely distinguish the individual from another are stored in association with each other, and
the searching unit searches the base sequence using SQL, which is a database language.

8. The base sequence determination device according to claim 7, wherein
the storage unit further includes:
an index that can be used for a prefix search, produced with respect to the base sequence stored in the database, and
the searching unit conducts the prefix search for the base sequence by referring to the index.

9. A base sequence determination method executed by an information processor including:
a control unit, and a storage unit, wherein
the storage unit includes:
a database that stores a plurality of base sequences derived from each of a plurality of parents and a plurality of descendants thereof having a genetic polymorphism,
the method comprising:
a step of connecting the base sequences by referring to the database included in the storage unit, using the genetic polymorphism as a marker,
wherein the step is executed by the control unit.

10. A base sequence determination method executed by an information processor including:
a control unit, and a storage unit, wherein
the storage unit includes:
a database that stores a plurality of base sequences derived from each of a plurality of parents and a plurality of descendants thereof having a genetic polymorphism,
the method comprising:
a searching step of searching, based on a target base sequence, which is the base sequence of the target parent, the base sequence to be connected to the target base sequence from the database included in the storage unit;
a detecting step of detecting the genetic polymorphism between the parents based on the base sequence searched at the searching step;
a verifying step of verifying whether a base sequence obtained from the identical parent is present in plural numbers in the base sequences searched at the searching step;
an examining step of examining, when the genetic polymorphism is detected at the detecting step and it is not verified at the verifying step that a base sequence obtained from the identical parent is present in plural numbers, a genotype of the descendants at a site at which the genetic polymorphism is detected based on the base sequence of the descendants searched at the searching step;
an assessing step of assessing whether the genotype of the descendants examined at the examining step and the genotype of the descendants that is previously examined match; and
an extending step of extending, when the genotypes are assessed to match each other at the assessing step, the target base sequence based on the base sequence of the parent searched at the searching step, and, when it is verified at the verifying step that a base sequence obtained from the identical parent is present in plural numbers, extends the target base sequences separately with respect to each of the base sequences of the identical parent searched at the searching step,
wherein the steps are executed by the control unit.

11. The base sequence determination method according to claim 10, wherein
the database is a relational database in which the base sequence of the individual and an individual-identifying information to uniquely distinguish the individual from another are stored in association with each other, and
the searching step includes searching the base sequence using SQL, which is a database language.

12. The base sequence determination method according to claim 11, wherein
the storage unit further includes:
an index that can be used for a prefix search, produced with respect to the base sequence stored in the database, and
the searching step includes conducting the prefix search for the base sequence by referring to the index.
